# EUROPEAN PATENT APPLICATION

(11) **EP 4 645 327 A1**
(43) Date of publication of application: **05.11.2025**
(21) Application number: 25173198.0
(22) Date of filing: 29.04.2025
(51) Int. Cl.: G16H 20/40, A61B 34/10, A61B 34/20, A61B 90/00, G16H 30/40

(54) **TECHNIQUES FOR GENERATING FOREIGN OBJECT EXTRACTION PLANS**

(30) Priority: 02.05.2024 US 202463641572 P
(71) Applicant: Stryker European Operations Limited, Carrigtwohill, Co. Cork T45 HX08 (IE)
(72) Inventor: De Massari, Daniele, 5614HV Eindhoven (NL); Willems, Nathalie, 1053DP Amsterdam (NL); Gibbons, Thomas Joseph, Manchester, M21 9WT (GB); Skellern, Barry, Preston, PR4 2TQ (GB)
(74) Representative: Schott, Jakob Valentin

(57) **Abstract**

Computer-implemented methods, systems, software, and techniques involve using an object-detection algorithm to detect a foreign object in a medical image of a target anatomy. The foreign object is non-native to the target anatomy. The pose of the foreign object in the medical image and a surgical plan is associated with the medical image. An interaction between the foreign object and the surgical plan is determined. Based on the interaction, an extraction plan is generated for removing the foreign object from the target anatomy.

## Description

### CROSS REFERENCE TO RELATED APPLICATIONS

The subject application claims priority to and all the benefits of United States Provisional Patent App. No. 63/641,572, filed May 2, 2024, the entire contents of which are hereby incorporated by reference.

### BACKGROUND

Planning for a surgical procedure, such as joint arthroplasty, may include the evaluation of a medical image or series of medical images of a target anatomy. The evaluation helps ensure the surgeons have a clear understanding of the surgical site and the size and position of implants to be installed at the site. The medical images can be acquired by using X-rays, computed tomography (CT) scans or magnetic resonance imaging (MRI) scans. The surgical plan is used intraoperatively to help guide the surgeon in carrying out the procedure according to the plan.

Planning for a surgical procedure, such as joint arthroplasty, may include the evaluation of a medical image or series of medical images of a target anatomy. The evaluation helps ensure the surgeons have a clear understanding of the surgical site and the size and position of implants to be installed at the site. The medical images can be acquired by using X-rays, computed tomography (CT) scans or magnetic resonance imaging (MRI) scans. The surgical plan is used intraoperatively to help guide the surgeon in carrying out the procedure according to the plan.

One complication to a surgical procedure is the presence of a non-native foreign object near or directly in the surgical site. For example, the foreign object may be a surgical screw, a broken surgical drill bit, a surgical sponge, or the like. In some cases, such foreign objects go completely undetected during surgery. Clearly, this outcome is not ideal for the patient or the surgeon. In other cases, discovery of such foreign objects occurs intraoperatively and comes as a surprise to surgeons during the procedure. As the patient is typically under anesthesia during the discovery, the surgeon must immediately formulate a plan to remove the foreign object. However, the surgeon often has no idea what the foreign object is, what surrounds the foreign object, or the optimal manner or tools to remove the foreign object. Therefore, the surgeon must manually improvise a plan for the removal of the foreign object. Removal of the foreign object may involve removing tissue or penetrating tissue to access the foreign object. During improvised removal of the foreign object, the surgeon may be unaware of the implications that the removal may have on the surgical plan or target site. It is possible that improvised removal of the foreign object can cause complications, such as damage to implant receiving surfaces, damage to sensitive structures (such as soft tissue or ligaments), or possible modification to the surgical plan. During the procedure, the surgeon is not afforded the luxury of updating or recreating a surgical plan that considers the implications of the foreign object. As such, manual improvised removal of foreign objects during a procedure creates an undesirable situation that may reduce the clinical outcome of the patient, prolong the duration of surgery, and add cost the procedure.

### SUMMARY

This Summary introduces a selection of concepts in a simplified form that are further described below in the Detailed Description. This Summary is not intended to limit the scope of the claimed subject matter nor identify key features or essential features of the claimed subject matter.

According to a first aspect, a computer-implemented method for evaluating a medical image of a target anatomy is provided. The method includes using an object-detection algorithm (object-detection algorithm) to detect a foreign object in the medical image. The foreign object is non-native to the target anatomy. The method further includes using the object-detection algorithm to determine a pose of the foreign object in the medical image. The method further includes associating a surgical plan with the medical image. The method further includes determining an interaction between the foreign object and the surgical plan. Based on the interaction, the method further includes generating an extraction plan for removing the foreign object from the target anatomy.

According to a second aspect, a surgical system is provided. The surgical system includes a surgical tool and a controller. The controller is configured to automatically evaluate, with an object-detection algorithm, a medical image to identify a foreign object in the medical image. The controller then determines, with the object-detection algorithm, a pose of the foreign object in the medical image. The controller then associates a surgical plan with the medical image and determines an interaction between the foreign object and the surgical plan. Finally, the controller generates, based on the interaction, an extraction plan to remove the foreign object with the surgical tool.

According to a third aspect, a non-transitory computer readable medium comprising instructions to automatically evaluate a medical image of a target anatomy is provided. The instructions, when executed by one or more processors, are configured to detect, with an object-detection algorithm, a foreign object in the medical image. The foreign object being non-native to the target anatomy. The instructions further determine, with the object-detection algorithm, a pose of the foreign object in the medical image and then associate a surgical plan with the medical image. The instructions further determine an interaction between the foreign object and the surgical plan and generate, based on the interaction, an extraction plan for removing the foreign object.

According to a fourth aspect, a computer-implemented method is provided. The method comprising automatically detecting, with an object-detection algorithm, a foreign object in a medical image of a target anatomy. In response to detecting the foreign object, the method further comprises automatically generating an extraction plan for removing the foreign object from the target anatomy.

According to a fifth aspect, a computer-implemented method is provided. The method comprising automatically detecting, with an object-detection algorithm, a foreign object in a medical image of a target anatomy. In response to detecting the foreign object, the method further comprises automatically generating an extraction path for removing the foreign object from the target anatomy.

According to a sixth aspect, a computer-implemented method is provided. The method comprising automatically detecting, with an object-detection algorithm, a foreign object in a medical image of a target anatomy. In response to detecting the foreign object, the method further comprises automatically suggesting a surgical workflow for removing the foreign object from the target anatomy.

According to a seventh aspect, a computer-implemented method is provided. The method comprising automatically detecting, with an object-detection algorithm, a foreign object in a medical image of a target anatomy. In response to detecting the foreign object, the method further comprises automatically suggesting a surgical tool setup for removing the foreign object from the target anatomy.

According to an eighth aspect, a computer-implemented method is provided. The method comprising automatically detecting, with an object-detection algorithm, a foreign object in a medical image of a target anatomy. In response to detecting the object, the method further comprises automatically suggesting a region of tissue to be removed from the target anatomy to facilitate removing the foreign object from the target anatomy, wherein the region of tissue includes the foreign object.

According to a ninth aspect, a computer-implemented method for guiding a surgeon to extract a foreign object from a target anatomy using a surgical system is provided. The surgical system comprising an extraction tool, a navigation system, a controller, and a display device. The computer-implemented method comprising detecting, with the controller, the foreign object in a medical image of the target anatomy. The method further comprises generating, with the controller, an extraction path for removing the foreign object with the extraction tool and tracking, with the navigation system, poses of the target anatomy and the extraction tool. The method further comprises implementing, with the controller, a graphical user interface on the display device. The graphical user interface is for producing representations of the target anatomy and the foreign object, a representation of the extraction path relative to the representations of the target anatomy and the foreign object. The graphical user interface further displays a representation of the extraction tool relative to the representations of the target anatomy, the foreign object, and the extraction path. A relative relationship between the representations of the extraction tool and the target anatomy is updated based on the navigation system tracking the poses of the target anatomy and the extraction tool. The graphical user interface further displays a graphical indicator for assisting the surgeon to follow the extraction path with the extraction tool for extracting the foreign object from the target anatomy.

Also provided are a surgical system configured to implement any of the aspects above, or a non-transitory computer-readable medium or a software program product configured to implement any of the aspects above.

Any of the above aspects can be utilized individually, or in combination.

Any of the above aspects can be utilized with any of the following implementations:
In one implementation, the computer-implemented method of generating the extraction plan for removing the foreign object further includes recommending an extraction tool for removing the foreign object.

In one implementation, the computer-implemented method further comprises of automatically determining, with the object-detection algorithm, a geometry of the foreign object and comparing the geometry of the foreign object to a database of known objects. In one implementation, the computer-implemented method further comprises of automatically identifying which of the known objects in the database is a best-fit match to the foreign object. In one implementation, the computer-implemented method further comprises of automatically suggesting an extraction tool for removing the foreign object, wherein the extraction tool is associated with the known object in the database that is the best-fit match to the foreign object. In one implementation, the computer-implemented method further comprises of automatically identifying that there exist no known objects in the database that are the best-fit match to the foreign object. In one implementation, the computer-implemented method further comprises of adding information related to the foreign object to the database.

In one implementation, the computer-implemented method further comprises the object detection algorithm feeding the medical image into a machine-learning model for classifying the foreign object. In one implementation, the computer-implemented method further comprises classifying the foreign object as a surgical implant. In one implementation, the computer-implemented method further comprises performing at least one of: identifying a type of the surgical implant; identifying a manufacturer of the surgical implant; identifying a bone ingrowth measurement of the surgical implant; and/or identifying how long the surgical implant has been implanted in the patient anatomy.

In one implementation, the computer-implemented method of generating the extraction plan further comprises of generating the extraction plan relative to the medical image. In one implementation, the computer-implemented method of generating the extraction plan relative to the medical image further comprises of generating a representation of an axis of the foreign object relative to the medical image for guiding a surgeon to remove the foreign object with an extraction tool relative to the axis.

In one implementation, the computer-implemented method of determining the interaction between the foreign object and the surgical plan further comprises of evaluating whether the extraction plan would disrupt the surgical plan. In one implementation, the computer-implemented method further comprises of the surgical plan relating to a surgical implant for a bone provided in the medical image. In one implementation, the computer-implemented method of evaluating whether the extraction plan would disrupt the surgical plan further comprises of determining whether the foreign object would impede installation or functionality of the surgical implant. In one implementation, the computer-implemented method further comprises of displaying, on a display device, an alert or notification in response to determining whether the foreign object would impede installation of the surgical implant. In one implementation, the computer-implemented method of generating the extraction plan for removing the foreign object further comprises of generating an extraction path for removing the foreign object. In one implementation, the computer-implemented method further comprises of evaluating whether the extraction path would impede installation of a surgical implant. In one implementation, the computer-implemented method further comprises of generating the extraction path for avoiding sensitive patient physiological structures.

In one implementation, the computer-implemented method of generating the extraction plan for removing the foreign object further comprises of suggesting a setup of a surgical tool for removing the foreign object. In one implementation, the computer-implemented method of generating the extraction plan further comprises of generating a suggested surgical workflow for removing the foreign object. In one implementation, the computer-implemented method of detecting, with the object-detection algorithm, the foreign object in the medical image further comprises of applying a radio density threshold for distinguishing the foreign object from surrounding patient physiological structures in the medical image.

### BRIEF DESCRIPTION OF THE DRAWINGS

Other advantages of the present disclosure will be readily appreciated, as the same becomes better understood by reference to the following detailed description when considered in connection with the accompanying drawings. Non-limiting and non-exhaustive embodiments of the present disclosure are described with reference to the following figures, wherein like numerals refer to like parts throughout the various views unless otherwise specified.
FIG. 1 is a perspective view of a surgical system, according to one implementation.
FIG. 2 is a functional block diagram carried out by components of the surgical system for detection and evaluation of a foreign object within a medical image relative to surgical plan, according to one implementation.
FIG. 3 illustrates a display presenting a medical image of a target anatomy with a surgical plan and a foreign object with an extraction path for removing the foreign object, according to one implementation.
FIG. 4A illustrates a display presenting a medical image and also presenting an extraction tool for removing the foreign object based on an extraction plan, wherein the extraction tool has crossed a boundary defined by an extraction plan, according to one implementation.
FIG. 4B illustrates the display presenting the medical image shown in FIG. 4A, wherein the extraction tool has exceeded a depth boundary defined by the extraction plan, according to one implementation.
FIG. 5 illustrates the display presenting a medical image and the extraction tool successfully removing the foreign object from the target anatomy, according to one implementation.
FIG. 6 is a flowchart for evaluating the medical image of the target anatomy, according to one implementation.
FIG. 7 is a flowchart for detecting the foreign object in the medical image with an object-detection algorithm, according to one implementation.
FIG. 8 is a flowchart for detecting the foreign object in the medical image with the object-detection algorithm using a machine-learning model, according to one implementation.
FIG. 9 is a flowchart for determining an interaction between the foreign object and the surgical plan, according to one implementation.
FIG. 10 is a flowchart for generating an extraction plan for removing the foreign object from the target anatomy, according to one implementation.

### DETAILED DESCRIPTION

In the following description, numerous specific details are set forth to provide a thorough understanding of the present invention. It will be apparent, however, to one having ordinary skill in the art that the specific details need not be employed and/or not be employed exactly as described to practice the present invention. In some instances, well-known materials or methods have not been described in detail to avoid obscuring the present invention.

### I. EXAMPLE SURGICAL SYSTEM

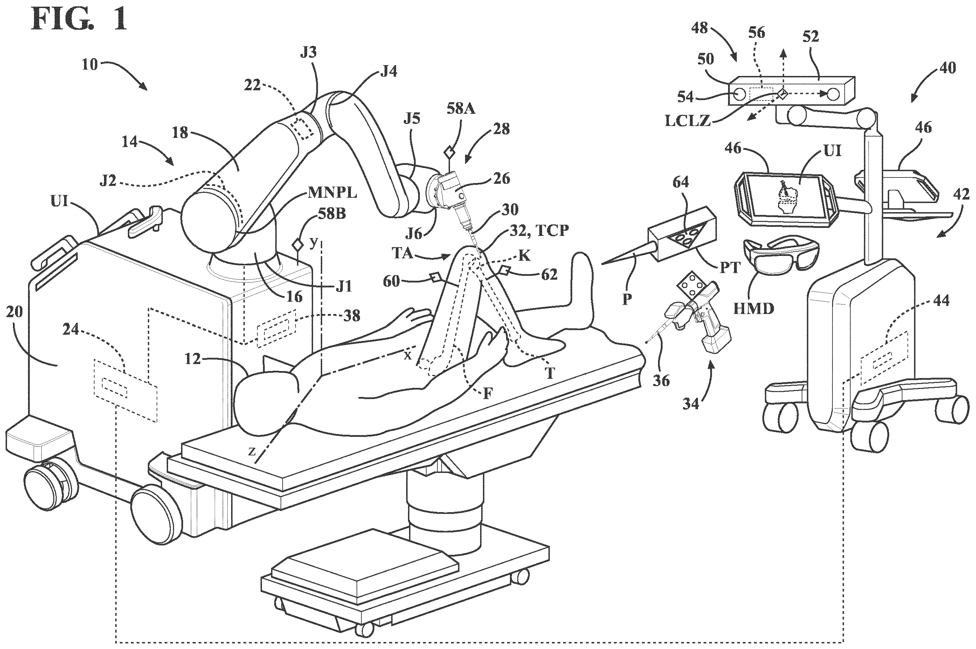

Referring to FIG. 1, an example surgical system 10 is illustrated. The system 10 is useful for treating a surgical site (referred to herein as a target anatomy TA) of a patient 12, such as treating bone or soft tissue. In FIG. 1, the patient 12 is undergoing a surgical procedure. The target anatomy TA in FIG. 1 includes a knee K of the patient 12. The surgical procedure may involve tissue removal or other forms of treatment. Treatment may include cutting, resecting, coagulating, lesioning the tissue, other in-situ tissue treatments, or the like. In some examples, the surgical procedure involves partial or total knee or hip replacement surgery, shoulder replacement surgery, spine surgery, endoscopy, or ankle surgery. In some examples, the system 10 is designed to cut away material to be replaced by surgical implants, such as hip and knee implants, including unicompartmental, bicompartmental, multicompartmental, or total knee implants. Some of these types of implants are shown in U.S. Patent Application Publication No. 2012/0330429, entitled, "Prosthetic Implant and Method of Implantation," the disclosure of which is hereby incorporated by reference. The system 10 and techniques disclosed herein may be used to perform other procedures, surgical or non-surgical, or may be used in industrial applications or other applications where robotic systems are utilized.

The system 10 can include a manipulator 14. The manipulator 14 has a base 16 and plurality of links 18. A manipulator cart 20 supports the manipulator 14 such that the manipulator 14 is fixed to the manipulator cart 20. The links 18 collectively form one or more arms of the manipulator 14. The manipulator 14 may have a serial arm configuration (as shown in FIG. 1), a parallel arm configuration, or any other suitable manipulator configuration. In other examples, more than one manipulator 14 may be utilized in a multiple arm configuration.

In the example shown in FIG. 1, the manipulator 14 comprises a plurality of joints J and a plurality of joint encoders 22 located at the joints J for determining position data of the joints. For simplicity, only one joint encoder 22 is illustrated in FIG. 1, although other joint encoders 22 may be similarly illustrated. The manipulator 14 according to one example has six joints J1-J6 implementing at least six-degrees of freedom (DOF) for the manipulator 14. However, the manipulator 14 may have any number of degrees of freedom and may have any suitable number of joints J and may have redundant joints.

The manipulator 14 need not require joint encoders 22 but may alternatively, or additionally, utilize motor encoders present on motors at each joint J. Also, the manipulator 14 need not require rotary joints, but may alternatively, or additionally, utilize one or more prismatic joints. Any suitable combination of joint types are contemplated.

The base 16 of the manipulator 14 is generally a portion of the manipulator 14 that provides a fixed reference coordinate system for other components of the manipulator 14 or the system 10 in general. Generally, the origin of a manipulator coordinate system MNPL is defined at the fixed reference of the base 16. The base 16 may be defined with respect to any suitable portion of the manipulator 14, such as one or more of the links 18. Alternatively, or additionally, the base 16 may be defined with respect to the manipulator cart 20, such as where the manipulator 14 is physically attached to the manipulator cart 20. In one example, the base 16 is defined at an intersection of the axes of joints J1 and J2. Thus, although joints J1 and J2 are moving components in reality, the intersection of the axes of joints J1 and J2 is nevertheless a virtual fixed reference pose, which provides both a fixed position and orientation reference and which does not move relative to the manipulator 14 and/or manipulator cart 20. In other examples, the manipulator 14 can be a hand-held manipulator where the base 16 is a base portion of a tool (e.g., a portion held free-hand by the user) and the tool tip is movable relative to the base portion. The base portion has a reference coordinate system that is tracked and the tool tip has a tool tip coordinate system that is computed relative to the reference coordinate system (e.g., via motor and/or joint encoders and forward kinematic calculations). Movement of the tool tip can be controlled to follow the path since its pose relative to the path can be determined.

The manipulator 14 and/or manipulator cart 20 house a manipulator controller 24, or other type of control unit. The manipulator controller 24 may comprise one or more computers, or any other suitable form of controller that directs the motion of the manipulator 14. The manipulator controller 24 may have a central processing unit (CPU) and/or other processors, memory (not shown), and storage (not shown). The manipulator controller 24 is loaded with software as described below. The processors could include one or more processors to control operation of the manipulator 14. The processors can be any type of microprocessor, multi-processor, and/or multi-core processing system. The manipulator controller 24 may additionally, or alternatively, comprise one or more microcontrollers, field programmable gate arrays, systems on a chip, discrete circuitry, and/or other suitable hardware, software, or firmware that is capable of carrying out the functions described herein. The term processor is not intended to limit any embodiment to a single processor. The manipulator 14 may also comprise a user interface UI with one or more displays and/or input devices (e.g., push buttons, keyboard, mouse, microphone (voice-activation), gesture control devices, touchscreens, etc.).

Any type of surgical tool 26 can be used with the system 10. The surgical tools 26 described herein may be any type of surgical instrument and the tool 26 may be coupled to the manipulator 14 or hand-held by the surgeon. The tool 26 may be passive or actively driven. The tool 26 may be a robotic hand-held tool. Examples of tools 26 include, but are not limited to: drills, routers, saws, routers, impactors, screwdrivers, awls, taps, extractors, gouges, pliers, scissors, retractors, and the like.

In one example, the tool 26 can couple to the manipulator 14 and is movable relative to the base 16 to interact with the anatomy in certain modes. In this case, the tool 26 is a physical and surgical tool and is or forms part of an end effector 28 supported by the manipulator 14 in certain embodiments. The tool 26 may be grasped by the user. One possible arrangement of the manipulator 14 and the tool 26 is described in U.S. Pat. No. 9,119,655, entitled, "Surgical Manipulator Capable of Controlling a Surgical Instrument in Multiple Modes," the disclosure of which is hereby incorporated by reference. The manipulator 14 and the tool 26 may be arranged in alternative configurations. The tool 26 can be like that shown in U.S. Patent Application Publication No. 2014/0276949, filed on Mar. 15, 2014, entitled, "End Effector of a Surgical Robotic Manipulator," hereby incorporated by reference.

The tool 26 can include an energy applicator 30 designed to contact and remove the tissue of the patient 12 at the surgical site. In one example, the energy applicator 30 is a bur 32. The bur 32 may be substantially spherical and comprise a spherical center, radius, and diameter. Alternatively, the energy applicator 30 may be a drill bit 36, a saw blade, an ultrasonic vibrating tip, or the like. The tool 26 and/or energy applicator 30 may comprise any geometric feature, e.g., perimeter, circumference, radius, diameter, width, length, volume, area, surface/plane, range of motion envelope (along any one or more axes), etc. The geometric feature may be considered to determine how to locate the tool 26 relative to the tissue at the surgical site to perform the desired treatment. In some of the embodiments described herein, a spherical bur having a tool center point (TCP) will be described for convenience and ease of illustration but is not intended to limit the tool 26 to any particular form.

The tool 26 may comprise a tool controller 38 to control operation of the tool 26, such as to control power to the tool (e.g., to a rotary motor of the tool 26), control movement of the tool 26, control irrigation/aspiration of the tool 26, and/or the like. The tool controller 38 may be in communication with the manipulator controller 24 or other components. The tool 26 may also comprise a user interface UI with one or more displays and/or input devices (e.g., push buttons, keyboard, mouse, microphone (voice-activation), gesture control devices, touchscreens, etc.). The tool controller 38 controls a state (position and/or orientation) of the tool 26 (e.g., the TCP) with respect to a coordinate system, such as the tool coordinate system MNPL. The tool controller 38 can control (linear or angular) velocity, acceleration, or other derivatives of motion of the tool 26.

The tool center point (TCP), in one example, is a predetermined reference point defined at the energy applicator 30. The TCP has a known, or able to be calculated (i.e., not necessarily static), pose relative to other coordinate systems. The geometry of the energy applicator 30 is known in or defined relative to a TCP coordinate system. The TCP may be located at the spherical center of the bur 32 of the tool 26 such that only one point is tracked. The TCP may be defined in various ways depending on the configuration of the energy applicator 30. The manipulator 14 could employ the joint/motor encoders, or any other non-encoder position sensing method, to enable a pose of the TCP to be determined. The manipulator 14 may use joint measurements to determine TCP pose and/or could employ techniques to measure TCP pose directly. The control of the tool 26 is not limited to a center point. For example, any suitable primitives, meshes, etc., can be used to represent the tool 26.

The system 10 further includes a navigation system 40. One example of the navigation system 40 is described in U.S. Pat. No. 9,008,757, filed on Sep. 24, 2013, entitled, "Navigation System Including Optical and Non-Optical Sensors," hereby incorporated by reference. The navigation system 40 tracks movement of various objects. Such objects include, for example, the manipulator 14, the tool 26 and the anatomy, e.g., knee K. The navigation system 40 tracks these objects to gather state information of each object with respect to a (navigation) localizer coordinate system LCLZ. Coordinates in the localizer coordinate system LCLZ may be transformed to the manipulator coordinate system MNPL, and/or vice-versa, using transformations.

The navigation system 40 includes a cart assembly 42 that houses a navigation controller 44, and/or other types of control units. A navigation user interface UI is in operative communication with the navigation controller 44. The navigation user interface UI includes one or more displays 46. The navigation system 40 is capable of displaying a graphical representation of the relative states of the tracked objects to the user using the one or more displays 46. The navigation user interface UI further comprises one or more input devices to input information into the navigation controller 44 or otherwise to select/control certain aspects of the navigation controller 44. Such input devices include interactive touchscreen displays. However, the input devices may include any one or more of push buttons, a keyboard, a mouse, a microphone (voice-activation), gesture control devices, and the like.

In some implementations, the display device may be configured as an extended reality device configured to execute any of the graphical functions described herein. The extended reality device can be implemented by a hand-held device (e.g., tablet or smart phone) or a head-mounted device HMD. The extended reality device may be configured to superimpose, overlay, or combine any of the described computer-generated graphics with real-world views to implement an extended reality, augmented reality, and/or mixed reality experience for the user. The real-world views may be views may be those acquired directly by the eyes of the user or may be a real-world video stream captured by one or more cameras of the extended reality device. When the head-mounted device HMD is utilized, the head-mounted device HMD may comprise a transparent lens or one or more display screens positioned directly in front of the eyes of the user to display the computer-generated graphics relative to the real-world views." The HMD used can be like that described in US Patent No. 10,499,997, entitled "Systems and Methods for Surgical Navigation", the entire contents of which are hereby incorporated by reference in their entirety.

The navigation system 40 also includes a navigation localizer 48 (referred to herein as a localizer) coupled to the navigation controller 44. In one example, the localizer 48 is an optical localizer and includes a camera unit 50. The camera unit 50 has an outer casing 52 that houses one or more optical sensors 54. The optical sensors 54 may be configured to detect infrared light, near-infrared light, and/or visible light. The localizer 48 may comprise its own localizer controller 56 and may further comprise a video camera VC or machine vision camera.

The navigation system 40 includes one or more trackers. In one example, the trackers include a pointer tracker PT, one or more manipulator trackers 58A, 58B, a first patient tracker 60, and a second patient tracker 62. In the illustrated example of FIG. 1, the manipulator tracker is firmly attached to the tool 26 (i.e., tracker 58A), the first patient tracker 60 is firmly affixed to a femur F of the patient 12, and the second patient tracker 62 is firmly affixed to a tibia T of the patient 12. In this example, the patient trackers 60, 62 are firmly affixed to sections of bone. The pointer tracker PT is firmly affixed to a pointer P used for registering the anatomy to the localizer coordinate system LCLZ. The manipulator tracker 58A, 58B may be affixed to any suitable component of the manipulator 14, in addition to, or other than the tool 26, such as the base 16 (i.e., tracker 58B), or any one or more links 18 of the manipulator 14. The trackers 58A, 58B, 60, 62, PT may be fixed to their respective components in any suitable manner. For example, the trackers may be rigidly fixed, flexibly connected (optical fiber), or not physically connected at all (ultrasound), as long as there is a suitable (supplemental) way to determine the relationship (measurement) of that respective tracker to the object that it is associated with.

Any one or more of the trackers may include active markers 64. The active markers 64 may include light emitting diodes (LEDs). Alternatively, the trackers 58A, 58B, 60, 62, PT may have passive markers, such as reflectors, which reflect light emitted from the camera unit 50. Other suitable markers not specifically described herein may be utilized.

The localizer 48 tracks the trackers 58A, 58B, 60, 62, PT to determine a state of each of the trackers 58A, 58B, 60, 62, PT, which correspond respectively to the state of the object respectively attached thereto. The localizer 48 may perform known triangulation techniques to determine the states of the trackers 58, 60, 62, PT, and associated objects. The localizer 48 provides the state of the trackers 58A, 58B, 60, 62, PT to the navigation controller 44. In one example, the navigation controller 44 determines and communicates the state the trackers 58A, 58B, 60, 62, PT to the manipulator controller 24. As used herein, the state of an object includes, but is not limited to, data that defines the position and/or orientation of the tracked object or equivalents/derivatives of the position and/or orientation. For example, the state may be a pose of the object, and may include linear velocity data, and/or angular velocity data, and the like.

The navigation controller 44 may comprise one or more computers, or any other suitable form of controller. Navigation controller 44 has a central processing unit (CPU) and/or other processors, memory (not shown), and storage (not shown). The processors can be any type of processor, microprocessor, or multi-processor system. The navigation controller 44 is loaded with software. The software, for example, converts the signals received from the localizer 48 into data representative of the position and orientation of the objects being tracked. The navigation controller 44 may additionally, or alternatively, comprise one or more microcontrollers, field programmable gate arrays, systems on a chip, discrete circuitry, and/or other suitable hardware, software, or firmware that is capable of carrying out the functions described herein. The term processor is not intended to limit any embodiment to a single processor.

Although one example of the navigation system 40 is shown that employs triangulation techniques to determine object states, the navigation system 40 may have any other suitable configuration for tracking the manipulator 14, tool 26, and/or the patient 12. In another example, the navigation system 40 and/or localizer 48 are ultrasound-based. For example, the navigation system 40 may comprise an ultrasound imaging device coupled to the navigation controller 44. The ultrasound imaging device images any of the aforementioned objects, e.g., the manipulator 14, the tool 26, and/or the patient 12, and generates state signals to the navigation controller 44 based on the ultrasound images. The ultrasound images may be 2-D, 3-D, or a combination of both. The navigation controller 44 may process the images in near real-time to determine states of the objects. The ultrasound imaging device may have any suitable configuration and may be different than the camera unit 50 as shown in FIG. 1.

In another example, the navigation system 40 and/or localizer 48 are radio frequency (RF)-based. For example, the navigation system 40 may comprise an RF transceiver coupled to the navigation controller 44. The manipulator 14, the tool 26, and/or the patient 12 may comprise RF emitters or transponders attached thereto. The RF emitters or transponders may be passive or actively energized. The RF transceiver transmits an RF tracking signal and generates state signals to the navigation controller 44 based on RF signals received from the RF emitters. The navigation controller 44 may analyze the received RF signals to associate relative states thereto. The RF signals may be of any suitable frequency. The RF transceiver may be positioned at any suitable location to track the objects using RF signals effectively. Furthermore, the RF emitters or transponders may have any suitable structural configuration that may be much different than the trackers 58A, 58B, 60, 62, PT shown in FIG. 1.

In yet another example, the navigation system 40 and/or localizer 48 are electromagnetically based. For example, the navigation system 40 may comprise an EM transceiver coupled to the navigation controller 44. The manipulator 14, the tool 26, and/or the patient 12 may comprise EM components attached thereto, such as any suitable magnetic tracker, electro-magnetic tracker, inductive tracker, or the like. The trackers may be passive or actively energized. The EM transceiver generates an EM field and generates state signals to the navigation controller 44 based upon EM signals received from the trackers. The navigation controller 44 may analyze the received EM signals to associate relative states thereto. Again, such navigation system 40 examples may have structural configurations that are different than the navigation system 40 configuration shown in FIG. 1.

The navigation system 40 may have any other suitable components or structure not specifically recited herein. Furthermore, any of the techniques, methods, and/or components described above with respect to the navigation system 40 shown may be implemented or provided for any of the other examples of the navigation system 40 described herein. For example, the navigation system 40 may utilize solely inertial tracking or any combination of tracking techniques, and may additionally or alternatively comprise, fiber optic-based tracking, machine-vision tracking, and the like.

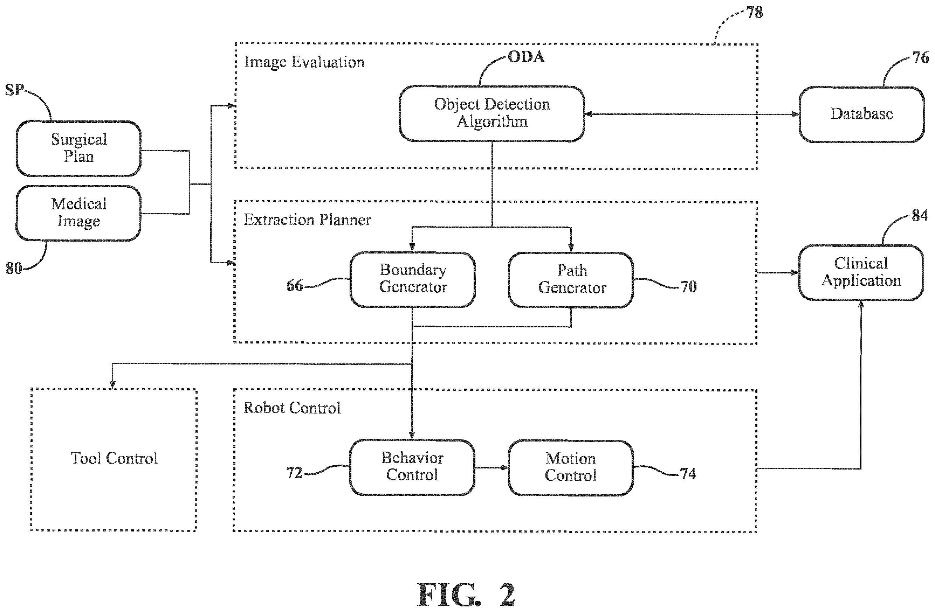

The system 10 further includes a control system that comprises, among other components, the manipulator controller 24, the navigation controller 44, and the tool controller 38. The control system further includes one or more software programs and software modules shown in FIG. 2. The software modules may be part of the program or programs that operate on the manipulator controller 24, navigation controller 44, tool controller 38, or any combination thereof, to process data to assist with control of the system 10. The software programs and/or modules include computer readable instructions stored in non-transitory memory on the manipulator controller 24, navigation controller 44, tool controller 38, or a combination thereof, to be executed by one or more processors of the controllers. The memory may be any suitable configuration of memory, such as RAM, nonvolatile memory, etc., and may be implemented locally or from a remote database. Additionally, software modules for prompting and/or communicating with the user may form part of the program or programs and may include instructions stored in memory on the manipulator controller 24, navigation controller 44, tool controller 38, or any combination thereof. The user may interact with any of the input devices of the navigation user interface UI or other user interface UI to communicate with the software modules. The user interface software may run on a separate device from the manipulator controller 24, navigation controller 44, and/or tool controller 38.

The control system may comprise any suitable configuration of input, output, and processing devices suitable for carrying out the functions and methods described herein. The control system may comprise the manipulator controller 24, the navigation controller 44, or the tool controller 38, or any combination thereof, or may comprise only one of these controllers. These controllers may communicate via a wired bus or communication network, via wireless communication, or otherwise. The control system may also be referred to as a controller. The control system may comprise one or more microcontrollers, field programmable gate arrays, systems on a chip, discrete circuitry, sensors, displays, user interfaces, indicators, and/or other suitable hardware, software, or firmware that is capable of carrying out the functions described herein.

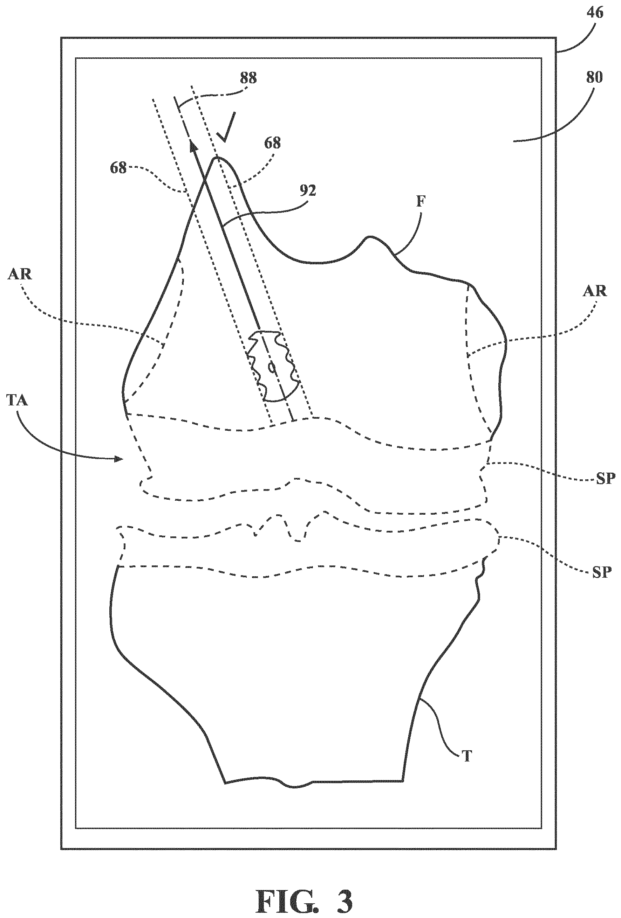

Referring to FIG. 2, the software employed by the control system can include a boundary generator 66. As shown in FIG. 3, the boundary generator 66 is a software program or module that generates a virtual boundary 68 for constraining movement and/or operation of the tool 26. The virtual boundary 68 may be one-dimensional, two-dimensional, three-dimensional, and may comprise a point, line, axis, trajectory, plane, or other shapes, including complex geometric shapes. In some embodiments, the virtual boundary 68 is a surface defined by a triangle mesh. Such virtual boundaries 68 may also be referred to as virtual objects. The virtual boundaries 68 may be defined with respect to an anatomical model, such as a 3-D bone model. In the example of FIG. 3, the virtual boundaries 68 are planar boundaries to constrain the tool 26 to remain within the virtual boundaries 68. The anatomical model is registered to the one or more patient trackers 60, 62 such that the virtual boundaries 68 become associated with the anatomical model. The virtual boundaries 68 may be implant-specific, e.g., defined based on a size, shape, volume, etc. of an implant and/or patient-specific, e.g., defined based on the patient's 12 anatomy. The virtual boundaries 68 may be boundaries that are created preoperatively, intra-operatively, or combinations thereof. In other words, the virtual boundaries 68 may be defined before the surgical procedure begins, during the surgical procedure (including during tissue removal), or combinations thereof. In any case, the control system obtains the virtual boundaries 68 by storing/retrieving the virtual boundaries 68 in/from memory, obtaining the virtual boundaries 68 from memory, creating the virtual boundaries 68 pre-operatively, creating the virtual boundaries 68 intra-operatively, or the like.

The manipulator controller 24 and/or the navigation controller 44 track the state of the tool 26 relative to the virtual boundaries 68. In one example, the state of the TCP is measured relative to the virtual boundaries 68 for purposes of determining haptic forces to be applied to a virtual rigid body model via a virtual simulation so that the tool 26 remains in a desired positional relationship to the virtual boundaries 68 (e.g., not moved beyond them). The results of the virtual simulation are commanded to the manipulator 14. The control system controls/positions the manipulator 14 in a manner that emulates the way a physical handpiece would respond in the presence of physical boundaries/barriers. The boundary generator 66 may be implemented on the manipulator controller 24. Alternatively, the boundary generator 66 may be implemented on other components, such as the navigation controller 44.

Referring to FIG. 2, a path generator 70 is another software program or module that can be run by the control system. In one example, the path generator 70 is run by the manipulator controller 24. The path generator 70 generates a tool path TP for the tool 26 to traverse, such as for removing sections of the anatomy to receive an implant. The tool path TP may comprise a plurality of path segments, or may comprise a single path segment. The path segments may be straight segments, curved segments, combinations thereof, or the like. The tool path TP may also be defined with respect to the anatomical model. The tool path TP may be implant-specific, e.g., defined based on a size, shape, volume, etc. of an implant and/or patient-specific, e.g., defined based on the patient's 12 anatomy.

FIG. 2 shows two additional software programs or modules that optionally can be run on the manipulator controller 24 and/or the navigation controller 44. One software module performs behavior control 72. Behavior control 72 is the process of computing data that indicates the next commanded position and/or orientation for the tool 26. In some cases, only the position of the TCP is output from the behavior control 72, while in other cases, the position and orientation of the tool 26 is output. Output from the boundary generator 66, the path generator 70, and a force/torque sensor may feed as inputs into the behavior control 72 to determine the next commanded position and/or orientation for the tool 26. The behavior control 72 may process these inputs, along with one or more virtual constraints described further below, to determine the commanded pose.

The second software module performs motion control 74. One aspect of motion control 74 is the control of the manipulator 14. The motion control 74 receives data defining the next commanded pose from the behavior control 72. Based on these data, the motion control 74 determines the next position of the joint angles of the joints J of the manipulator 14 (e.g., via inverse kinematics and Jacobian calculators) so that the manipulator 14 is able to position the tool 26 as commanded by the behavior control 72, e.g., at the commanded pose. In other words, the motion control 74 processes the commanded pose, which may be defined in Cartesian space, into joint angles of the manipulator 14, so that the manipulator controller 24 can command the joint motors accordingly, to move the joints J of the manipulator 14 to commanded joint angles corresponding to the commanded pose of the tool 26. In one version, the motion control 74 regulates the joint angle of each joint J and continually adjusts the torque that each joint motor outputs to, as closely as possible, ensure that the joint motor drives the associated joint J to the commanded joint angle.

The boundary generator 66, path generator 70, behavior control 72, and motion control 74 may be sub-sets of a software program. Alternatively, each may be software programs that operate separately and/or independently in any combination thereof. The term "software program" is used herein to describe the computer-executable instructions that are configured to carry out the various capabilities of the technical solutions described. For simplicity, the term "software program" is intended to encompass, at least, any one or more of the boundary generator 66, path generator 70, behavior control 72, and/or motion control 74. The software program can be implemented on the manipulator controller 24, navigation controller 44, or any combination thereof, or may be implemented in any suitable manner by the control system. Other aspects of the control system will be described below in relation to an improved techniques for foreign object detection, evaluation, and surgical guidance related to the same.

### II. DETECTION OF FOREIGN OBJECT

Described in this section are techniques for detecting a foreign object 86 in a medical image 80 and performing advanced techniques for planning removal of the foreign object 86 and optionally providing surgical guidance for removal of the foreign object 86. Any of the components, capabilities, features, or configurations of the system 10 described above can be utilized for the techniques described herein. Any of the aspects described in the detailed description involving evaluation of the medical image 80 may be visualized to a user on a display device or may be performed without any visualization to the user. Also, any aspects involving evaluation of the medical image 80 may be performed automatically or may include manual user input at certain stages.

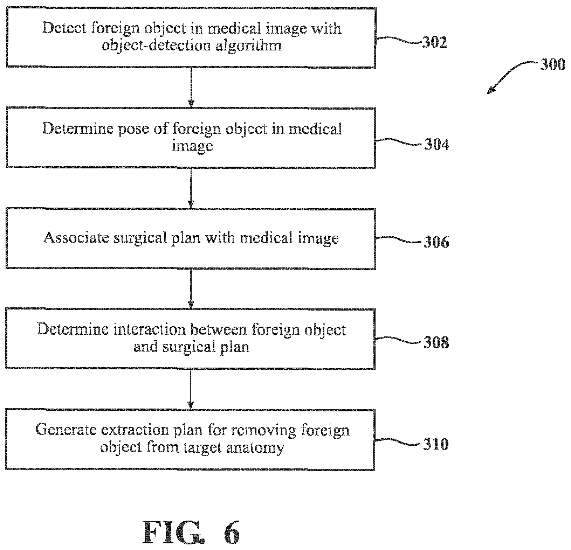

FIG. 6 shows a flowchart of an example computer-implemented method 300 for automatically evaluating the medical image 80 of the target anatomy TA. As shown in the flowchart, the first step 302 includes detecting, with an object-detection algorithm ODA, a foreign object 86 in the medical image 80, where the foreign object 86 is non-native to the target anatomy TA. The second step 304 includes determining, with the object-detection algorithm ODA, a pose of the foreign object 86 in the medical image 80. These steps 302, 304 are discussed in further detail below.

The foreign object(s) 86 that are detected by the object-detection algorithm ODA can include any foreign body object that is located within the target anatomy TA in the medical image 80. The foreign object 86 is non-native to the patient 12. In other words, the foreign object 86 is not an organically growing structure of the patient 12. The foreign object 86 is often unintentionally retained within the target site. Examples of foreign objects, include, but are not limited to: tools (e.g., needle or markers), tool fragments (e.g., broken drills, broken surgical screws or staples), anatomical fragments, implants (e.g., surgical screw), implant fragments, implantable medical devices, internal fixation devices (such as plates, intramedullary rods, wires), gauze, surgical fabric, pads, surgical sponges, and the like.

Referring back to FIG. 2, the object-detection algorithm ODA can be a software program run on any computing system that facilitates image evaluation 78. The computing system can be any one or more of those described above, such as the manipulator controller 24 and/or the navigation controller 44. In some cases, the computing system can be a separate device (e.g., laptop or desktop PC) that is not in the operating room, or a computing system located on a remote server. Any of the described controller(s) can be used to implement the object-detection algorithm ODA and techniques described herein. For simplicity, where appropriate, the computing system that operates the functionality of the object detection algorithm ODA and other features will be referred to as a "controller."

The object-detection algorithm ODA aims to identify and locate any one or more foreign objects 86 and/or other objects within the medical image 80. For example, FIG. 3 illustrates the display 46 showing the medical image 80 of the target anatomy TA according to one implementation. The medical image 80 includes the patient's 12 knee including the foreign object 86 found in the target anatomy TA at the time of image acquisition. The medical image 80 analyzed by the object-detection algorithm ODA may be acquired preoperatively or intraoperatively using any appropriate scanning system (e.g., C-arm, fluoroscopy scanner, CT scanner, X-ray scanner, etc.). The medical image 80 can also include a 2D or 3D image set. The object-detection algorithm ODA receives and analyzes the medical image 80 to identify foreign objects 86 and/or other objects using any one or more following techniques utilized individually or in combination: image segmentation, shape modeling, statistical shape modeling, active shape modeling, atlas mapping, bounding boxes, pattern recognition, edge detection, Hough transforms, templating, gradient matching, metal artifact reduction, and the like. The object-detection algorithm ODA can utilize any number of machine learning models, such as neural networks, convolutional neural networks, deep learning models, and the like. The machine learning models can be trained on datasets of prior medical images, prior foreign objects, and/or any prior object found in medical images. The object-detection algorithm ODA helps automate image evaluation in a manner that cannot be practically performed using manual methods.

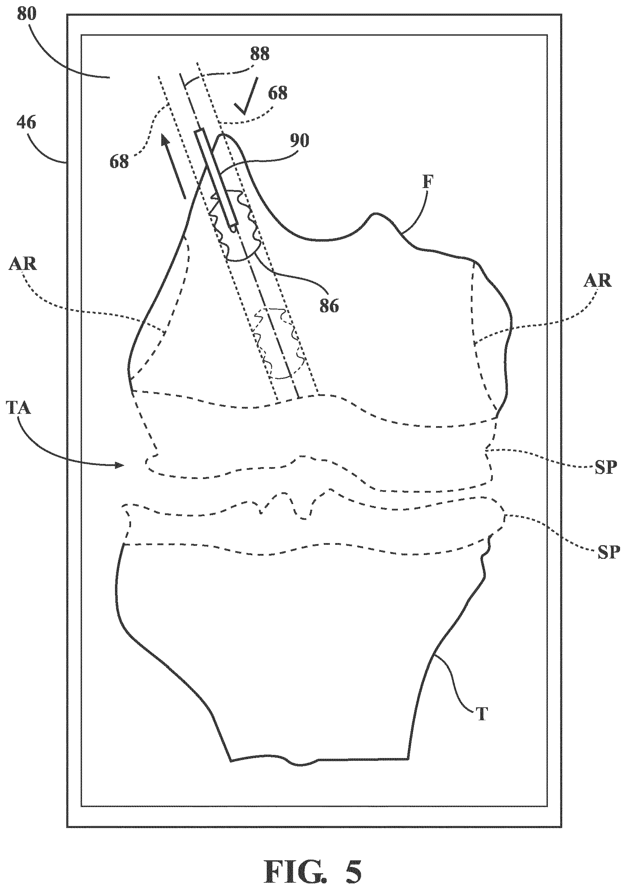

As shown in FIG. 3, the display 46 shows the medical image 80 of a representation of the target anatomy TA. The target anatomy TA in the medical image 80 includes the patient's 12 knee K (shown as the patient's 12 femur F and tibia T) and the foreign object 86. The foreign object 86 is non-native to the patient's 12 target anatomy TA and is represented in FIGS. 3-5 as an implanted surgical screw. However, as described, the foreign object 86 may be other types.

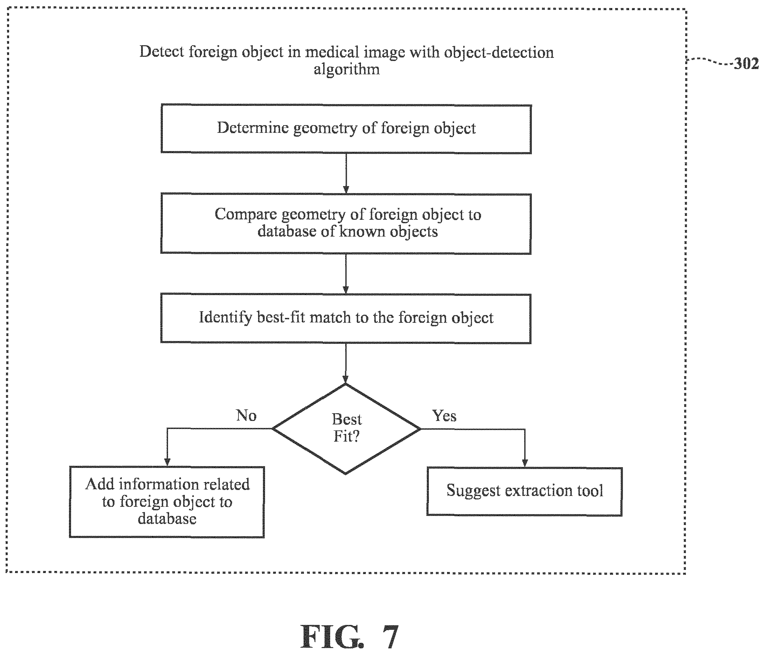

FIG. 7 shows an example computer-implemented method for detecting the foreign object 86 in the medical image 80 with an object-detection algorithm ODA. After receiving the medical image 80, the object-detection algorithm ODA can determine a geometry of the foreign object 86 and the controller can compare the geometry to a database 76 of known objects. The comparison is done to determine if there exists a best-fit match between the foreign object 86 in the medical image 80 and a known object in the database 76. If the foreign object 86 found in the medical image 80 is found to have the best-fit match available in the database 76, that will help determine important attributes about the foreign object 86. For example, if the foreign object 86 is a surgical screw, such attributes may include the surgical screw's orientation, geometry, material composition, thread design, diameter, length, pitch, and functional considerations. Some material compositions may include stainless steel or titanium. Thread design may help determine if the foreign object 86 is a cortical bone screw, which are designed for dense outer bone layers, or cancellous bone screws, which are suitable for spongy inner bones. The diameter of the foreign object 86 may help determine the minimal hole size required for accommodating the screw in the bone and the tool 26 used for its installation. The length of the surgical screw may help determine how far into the bone the surgical screw extends. The pitch may help determine how far the surgical screw travels with each 360-degree rotation. More threads may help determine how much resistance there will be in possibly removing the surgical screw. In other words, if there exists the best-fit match within the database 76, it will help identify the foreign object 86, its attributes, and help strategize next steps for addressing the foreign object 86.

However, in some instances, the object-detection algorithm ODA may not be able to find the best-fit match between the foreign object 86 in the medical image 80 and the database 76 of known objects. In response to finding no best-fit match, the controller will add information related to the foreign object 86 to the database 76. This will ensure that subsequent encounters with the foreign object 86 are more predictable and ultimately successful. The information that the object-detection algorithm ODA adds to the database 76 may include the attributes discussed above but may also include other attributes not mentioned.

Additionally, the object-detection algorithm ODA may identify a pose or geometrical features of the foreign object 86. The pose of the foreign object 86 may include its spatial configuration within the medical image 80 including an axis 88 (shown in FIG. 3) of the foreign object 86, its geometry, and its orientation within the medical image 80. The axis 88 of the foreign object 86 may provide meaningful insight into how the foreign object 86 was installed in the patient's 12 bone including the tool 26 used for the installation. The foreign object's 86 axis 88 may provide a reference line or centerline for the foreign object's 86 placement and orientation in the bone. For example, if the foreign object 86 is a surgical screw, the axis 88 can provide insight into the surgical screw's function, stability, and biomechanics. The axis 88 may also provide useful information regarding the removal of the foreign object 86, which is discussed in detail in subsequent sections. As an additional example, if the foreign object 86 is a broken drill bit that has broken up into multiple pieces, the object-detection algorithm ODA may identify the pose or geometrical features of each individual fragment of the broken drill bit. The geometrical features can be detected using any of the image detection algorithms or machine learning models described above.

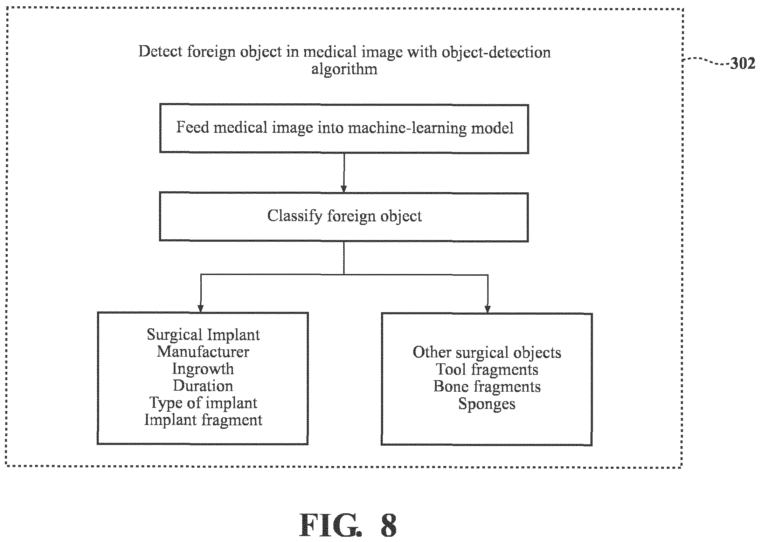

FIG. 8 is an example computer-implemented method for detecting the foreign object 86 in the medical image 80 with the object-detection algorithm ODA using a machine-learning model. The object-detection algorithm ODA may help identify the foreign object 104 by feeding the medical image 80 into the machine-learning model for classifying the foreign object 86. The machine-learning model is a computer program designed to recognize patterns in data and/or make predictions. The machine-learning model may be specifically designed to analyze medical images. These models learn from image data to perform such tasks as image classification, object detection, and/or segmentation. Image classification includes assigning labels to what the machine-learning model finds in an image. For example, the machine-learning model may analyze the medical image 80 shown in FIG. 3 and classify the foreign object 86 as a surgical implant such as a surgical screw. Object detection may include locating and identifying specific objects within an image 80. For example, the machine-learning model may analyze the medical image 80 in FIG. 3 and determine the location of the foreign object 86 relative to other objects within the medical image 80. Segmentation is a task that can be done by the machine-learning model to separate an image into meaningful regions. For example, the machine-learning model may identify a region in the medical image 80 shown in FIG. 3 showing a sensitive patient physiological structure that may need to be avoided.
The sensitive patient physiological structure may be deemed an avoidance region AR, which is shown in FIG. 3. Some sensitive structures may include arteries, veins, nerves, spinal cord, tendons, and/or ligaments. Avoiding such structures can prevent injuring the patient 12. Therefore, the machine-learning model classifying the foreign object 86 and the surrounding avoidance region AR within the medical image 80 can help prevent potential patient injuries and help ensure a successful clinical outcome.

Along with classifying the foreign object 86 as a surgical implant, the machine-learning model may also identify a manufacturer of the surgical implant, bone ingrowth measurement of the surgical implant, and/or how long the surgical implant has been implanted in the target anatomy TA, which also shown in FIG. 8. In addition to the attributes discussed above, the manufacturer of the surgical implant may also help determine the quality and safety assurance of the surgical implant. The manufacturer may also help determine traceability and recalls, which may uncover safety concerns associated with the particular surgical implant. It may also help determine the longevity and durability of a given surgical implant by having access to the manufacturer's specifications which may help estimate how long the surgical implant should last. Bone ingrowth around the surgical implant may provide important information relating to the surgical implant's lifespan and stability. Bone ingrowth may help determine how well the implant integrates with the patient's 12 surrounding bone tissue. Bone ingrowth also helps provide information regarding the fixation strength of the surgical implant. Bone attachment ensures that the surgical implant remains in place and is less likely to wear out or require early replacement. The machine-learning model may also classify objects within the medical image 80 as being other surgical objects. For example, tool fragments, bone fragments, or surgical sponges.

The object-detection algorithm ODA may also be used to identify the foreign object 86 within the medical image 80 by applying a radio density threshold for distinguishing the foreign object from surrounding patient physiological structures in the medical image. Radio density thresholding is a technique used in medical imaging to distinguish between different types of tissues or materials based on their radiodensity, which is a measure of how much they attenuate or absorb x-rays. The radio density threshold is applied to the medical image 80 to separate the objects of interest based on their radio densities. If the foreign object 86 is made of out metal, the foreign object 86 will have a high contrast when exposed to a radio density threshold, which allows the object-detection algorithm ODA to locate the foreign object 86 within the medical image 80.

### III. ASSOCIATING SURGICAL PLAN WITH MEDICAL IMAGE

Described herein are techniques for associating a surgical plan with the medical image 80 and to evaluate potential implications of the foreign object 86 of the surgical plan. The techniques described herein can determine how the foreign object 86 affects the surgical plan and potentially generating modifications to the surgical plan SP and/or the plan for extracting the foreign object 86 to mitigate complications during surgery. Any of the aspects described in the detailed description involving evaluation of the surgical plan SP relative to the medical image 80 may be visualized to a user on a display device or may be performed without any visualization to the user. Also, any aspects involving evaluation of the surgical plan SP relative to the medical image 80 may be performed automatically or may include manual user input at certain stages.

Continuing with FIG. 6, step 306 of the method 300 includes associating the surgical plan SP with the medical image 80. Aspects of the surgical plan SP are illustrated in FIG. 3. The surgical plan involves planning and/or visualizing aspects of a surgical procedure prior to an actual procedure. The surgical plan helps evaluate the feasibility, safety, and efficiency of a given surgical procedure and assists with proper execution of the procedure while minimizing complications. The surgical plan SP can be preoperatively and/or intraoperatively determined. The surgical plan SP can outline surgical steps or any sequence of actions during surgery. Such steps may include but are not limited to: operating room setup, anatomy registration, anatomy preparation, post-operative evaluation, or the like. Surgical actions that can be part of the surgical plan can include but are not limited to: performing incisions, tissue removal, tissue dissection, implant placement, and/or closure. The surgical plan SP can include any generic or patient-specific parameters, information, or aspects, including but not limited to: procedure type, operative side definition, definition of target site, patient information, bone models, implant models, target trajectories, cut planes, virtual boundaries, cutting paths, surfaces of the target anatomy TA that should be removed and/or remain to receive an implant, implant size, implant pose, implant type, bone mineral density, critical structure location, landmark location, osteophyte location, tool definition or operating parameters, what/how/when certain tools will be used, ligament location, ligament balancing information, biomechanical data (e.g., joint range of motion), manipulator modes of operation, joint parameters, joint gap size, and the like. Other aspects of a surgical plan are contemplated and such aspects will vary depending on the type of procedure to be performed.

Any of these details of the surgical plan SP can be provided as a visual feature that can be combined with, or overlayed onto the medical image 80 for evaluation. The surgical plan SP can include image data and/or non-image data. The surgical plan SP can be defined relative to patient imaging data, (such as a CT, X-ray, etc.) or the medical image 80 itself. In other examples, the surgical plan SP is defined relative to a virtual model of the target anatomy TA. The virtual model can be derived from segmentation of the medical imaging data or the medical image 80 itself. The surgical plan SP can include virtual implant components combined with the virtual model of the target anatomy TA. Measurements related to the surgical plan can be defined as data that may or may not have visual aspects.

FIG. 3 illustrates the display 46 showing an example surgical plan SP in the medical image 80 with a dotted line. The surgical plan SP in FIG. 3 shows a representation of a total knee replacement. The regions identified by the surgical plan SP are regions to be removed for receipt of knee joint implants (e.g., tibial component and femoral component). These regions can be defined by virtual boundaries. To ensure that the surgical plan SP proves to be successful and without intraoperative complications, the foreign object 86, its location and pose should be considered in the planning phase. Interaction between the surgical plan SP and the foreign object 86 is therefore evaluated using the techniques described herein. This interaction will be described in the subsequent section.

### IV. INTERACTION OF FOREIGN OBJECT AND SURGICAL PLAN

Referring back to FIG. 6, step 308 includes determining an interaction between the foreign object 86 and the surgical plan SP. This step 308 helps in understanding how the surgical plan SP is impacted or disrupted by the presence of the foreign object 86 located in the target anatomy TA.

Determining an interaction between the foreign object 86 and the surgical plan SP can be performed in many ways. The interaction can be a visual comparison and/or a data comparison. For visual comparison, visual aspects of the surgical plan SP can be imported into, transformed to, superimposed, templated, or otherwise combined with the medical image 80. For example, in FIG. 3, the surgical plan SP includes regions (e.g., defined by virtual boundaries) to be removed for receipt of knee joint implants and the parameters of these regions are visually provided on the medical image 80. The surgical plan SP also includes one or more avoidance regions AR, which may be virtual boundaries related to sensitive anatomical structures to be avoided during surgery (such as ligaments or veins). The foreign object 86 is already visually captured in the medical image 80 and hence can be readily compared to visual aspects of the surgical plan SP. Visual measurements or parameters of the foreign object 86 can also be determined. The medical image 80, foreign object 86, and the surgical plan SP can be represented relative to one or more virtual model of the target anatomy TA or relative to native patient imaging data. In this case, interaction between the foreign object 86 and surgical plan SP is detected. The foreign object 86 is detected above, and proximity to, the region to be removed for receipt of the tibial component. The foreign object 86 is also detected in between the avoidance regions AR. Visual interaction need not require a user visually observe the interaction. Rather, the controller can assess the visual interaction between the surgical plan SP and the foreign object 86 automatically and unbeknownst to the user.

Visually comparing interaction between the foreign object 86, target anatomy TA, medical image 80 and surgical plan SP can be performed by the controller, the object-detection algorithm ODA, or any other dedicated algorithm configured to compare interaction between visual features. Hence, any one or more following techniques can be utilized individually or in combination to visually compare interaction: image segmentation, shape modeling, statistical shape modeling, active shape modeling, atlas mapping, bounding boxes, pattern recognition, edge detection, Hough transforms, templating, gradient matching, machine learning models, such as neural networks, convolutional neural networks, deep learning models, and the like. The machine learning models can be trained on datasets of prior surgical plans, prior medical images, prior foreign objects, and/or any prior object found in medical images. These techniques can automate visual comparison between the foreign object 86, target anatomy TA, medical image 80 and surgical plan SP in a manner that cannot be practically performed using manual methods.

In other implementations, the interaction can be evaluated using data comparison. Data comparison can be performed in addition to or instead of visual comparison. For data comparison, parameters or measurements of the surgical plan SP can be imported into, transformed to, or otherwise combined with parameters or measurements of the medical image 80. For example, in FIG. 3, the regions to be removed for receipt of knee joint implants and the avoidance regions AR identified by the surgical plan SP can be defined by their measurements relative to features of the target site. Measurements of the surgical plan SP may include the coordinates, length, width, depth, orientation, position of any aspect of these surgical plan SP. The medical image 80 can be defined by its size, border size, resolution, or the like. The size and pose of the target site relative to the medical image 80 can be measured. Measurements or parameters of the foreign object 86 can be defined by its respective data. The respective parameters and/or measurements of the foreign object 86, target anatomy TA, medical image 80 and surgical plan SP can be combined into a common coordinate system, such as the coordinate system of the medical image 80, the coordinate system of the surgical plan SP, or any arbitrary coordinate system. Comparing interaction between the foreign object 86, target anatomy TA, medical image 80 and surgical plan SP based on data can be performed by the controller, the object-detection algorithm ODA, or any other algorithm dedicated to comparing such data to determine the respective interactions.

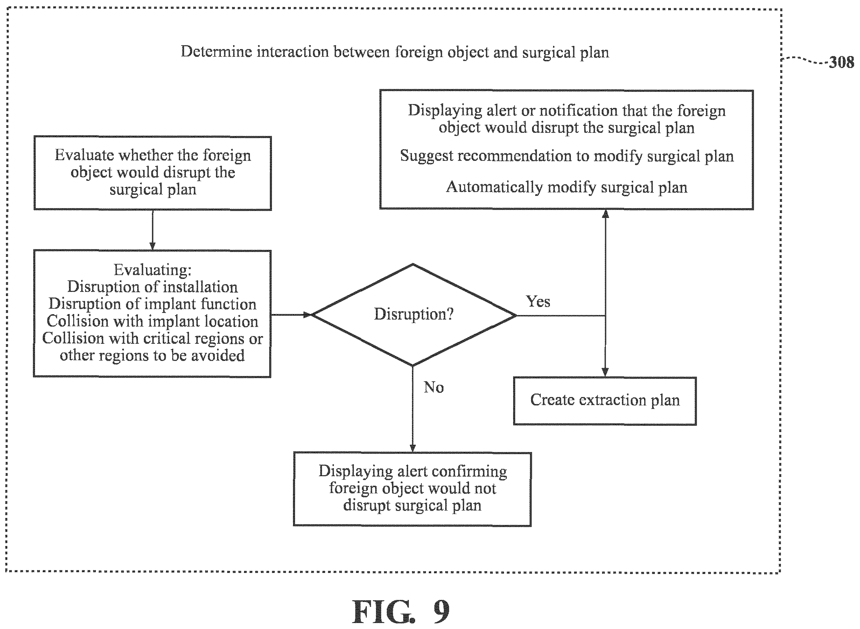

Examples techniques for determining interaction between the foreign object and surgical plan are further defined in FIG. 9. Based on the output of comparing the foreign object 86, target anatomy TA, medical image 80 and surgical plan SP, the controller can determine whether the foreign object 86 would disrupt or potentially disrupt the surgical plan SP. Disruption to the surgical plan SP can be evaluated in numerous ways. Any of the described aspects of the foreign object 86, target anatomy TA, medical image 80 and surgical plan SP can play a role in the determination and can include factors such as specific implant (type/size/location), functionality of implant, implant life, and steps or complexity of the surgical procedure. Also, the foreign object's 86 precise location, stability, and the patient's 12 overall health including bone health are factors that may be considered during the determination. Additionally, the controller can detect any one or more of: disruption or potential disruption of the foreign object 86 on implant installation; disruption or potential disruption of the foreign object 86 on implant function; collision or potential collision of the foreign object 86 on planned implant location; collision or potential collision of the foreign object 86 with critical or sensitive anatomical structures; or the like. Such conditions can be compared to thresholds, such as distance thresholds. For example, a potential collision may be detected if the foreign object 86 is within 5mm of any avoidance region AR or planned implant location. If such conditions are detected, the surgical plan SP would either need to be altered or the foreign object 86 would need to be removed from the target anatomy TA. For example, if the foreign object 86 would impede installation of the surgical implant, the aspects associated with installing the surgical implant may be changed. This may include changing the pathway taken to install the surgical implant, changing the incision location as part of the installation, or possibly changing the shape of the surgical implant.

In response to detecting disruption to the surgical plan SP, the controller can perform many functions. In one example, as will be described below, the controller generates an extraction plan for removing the foreign object 86. Additionally, or alternatively, the controller can generate an alert or notification notifying that the foreign object 86 disrupts the surgical plan SP, with details regarding the disruption for review by the surgeon. The notification can be displayed on the display 46 (shown in FIG. 1) or shown on the head-mounted display HMD (also shown in FIG. 1). In other scenarios, the controller can automatically modify the surgical plan SP, which can be reviewed by the surgeon. Alternatively, the controller can automatically suggest a modification to the surgical plan SP. The suggestion can be displayed on the display 46 or HMD display.

It is possible that the controller determines that the foreign object 86 does not affect the surgical plan SP. For example, the surgical plan SP may depict a total knee replacement (e.g., as shown in FIG. 3) and the foreign object 86 is an existing implant such as a surgical screw. It may be possible that the surgical plan SP is unaffected by the presence of the surgical screw in the target anatomy TA. A total knee replacement procedure typically involves replacing a damaged joint with new knee implant components (femoral and tibial components) in precise locations. If the surgical screw is not in the path of installation of the total knee implant components or would not compromise the longevity and lifespan of the total knee implant components, it may be possible that the surgical plan SP does not need to be altered despite the presence of the surgical screw. Again, the controller can generate an alert or notification notifying that the foreign object 86 would not disrupt the surgical plan SP, with a report providing a reasoned analysis describing why there would be no disruption for review by the surgeon.

### V. EXTRACTION PLAN

Described herein are techniques for generating an extraction plan for removing the foreign object 86. Referring back to FIG. 6, step 310 includes generating, based on the interaction between the foreign object 86 and the surgical plan SP, an extraction plan for removing the foreign object 86 from the target anatomy TA. The controller is configured to automatically generate the extraction plan. In some cases, the controller can generate the extraction plan in response to detecting a disruption or potential disruption to the surgical plan SP. However, it is contemplated that the controller can generate the extraction plan regardless of the surgical plan SP. Namely, a surgeon may desire that the foreign object 86 needs to be extracted even if the foreign object 86 does not disrupt the surgical plan SP. In such cases, the techniques described herein can be utilized as a means to generate a detailed extraction plan for removing the foreign object 86 from any target anatomy TA, with or without a surgical plan SP. Also, it is possible to generate the extraction plan after execution of the surgical plan SP. Further, generation of the extraction plan need not require a user visually observe the extraction plan. Rather, the controller can generate the extraction plan automatically and unbeknownst to the user.

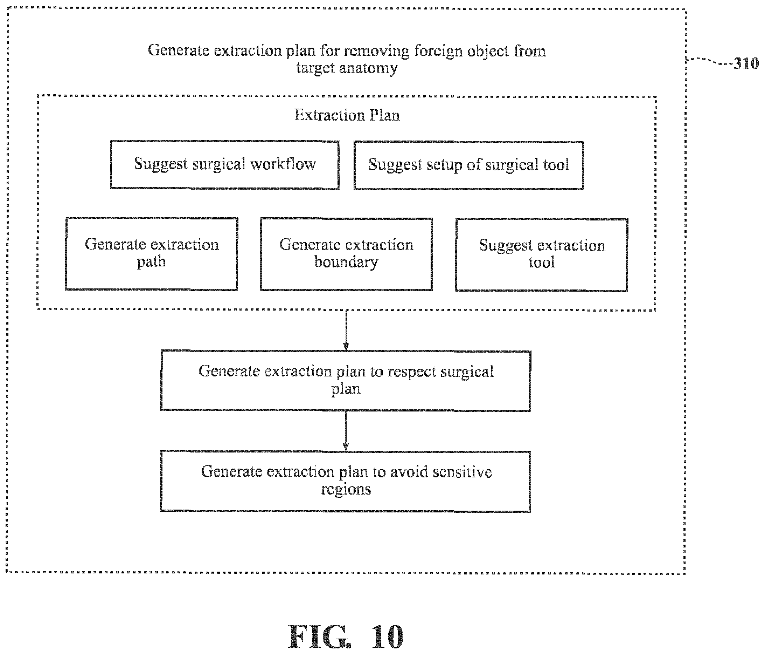

FIG. 10 outlines example techniques for generating an extraction plan for removing the foreign object 86 from the target anatomy TA. The extraction plan can include a variety of planned features, recommendations, aspects, or details. For example, the extraction plan can include a recommendation for an extraction tool 90 for removing the foreign object 86. The extraction tool 90 can be any type of surgical tool, including any of the surgical tools 26 described above that can be used with the system 10. As such, the extraction tool 90 can include the manipulator 14 and/or the surgical instrument coupled to the manipulator 14. The extraction tool 90 can be hand-held by the surgeon. The extraction tool 90 may be passive or actively driven. The extraction tool 90 may be controlled via gestures and/or voice commands. The head-mounted device HMD may also assist in providing a means for controlling the extraction tool 90 and for changing the extraction path 92 intraoperatively. For example, changing the extraction path intra-operatively may occur if after removing or repositioning the tissue surrounding the foreign object 86, the user may decide to alter the extraction path 92 that may result in a better outcome than the original extraction path 92. The head-mounted device HMD may also assist the user in confirming that the tissue surrounding the foreign object 86 has successfully been moved to accommodate the extraction path 92. The extraction tool 90 may be a robotic hand-held tool. Examples of extraction tools 90 include, but are not limited to: drills, routers, saws, routers, impactors, screwdrivers, awls, taps, extractors, gouges, pliers, scissors, grasper, clamp, retractors, and the like.

The controller is configured to recommend the extraction tool 90 as part of the extraction plan. In some cases, the controller can make such recommendations based on predetermined data. As discussed above, if there exists a best-fit match to the foreign object 86 in the database 76, the extraction tool 90 may be associated with the foreign object 86 be listed in the database 76. If the foreign object's 86 best-fit match is found in the database 76, then the foreign object 86 has been seen before and catalogued in the database 76 along with many of the attributes of the foreign object 86 previously mentioned in addition to the required extraction tool 90. For example, if the foreign object 86 is a surgical screw and there exists a best-fit match to the surgical screw within the database 76, the extraction tool 90 may be listed in the database 76, which may include the exact drill bit size or tool to use for removing the surgical screw from the target anatomy TA. The extraction tool 90 could be the drill 34 with the drill bit 36 as shown in FIG. 1, as an example. Knowing the exact extraction tool 90 to use helps plan the foreign object removal ahead of time and assists to ensure that the foreign object 86 can be removed safely and successfully.

In other scenarios, if the extraction tool 90 and/or foreign object 86 are not stored in a database, the controller can automatically suggest a best-fit recommended extraction tool using any appropriate algorithm for recommending extraction tools 90. For example, the controller or object-detection algorithm ODA can utilize any number of machine learning models, such as neural networks, convolutional neural networks, deep learning models, and the like. The machine learning models can be specifically trained on datasets of prior foreign objects and extraction tools. For example, the inputs to the machine learning model(s) can be the detected foreign object 86 parameters and the medical image 80. Based on the inputs, the machine learning model(s) can recommend an extraction tool that is best suited for removal of the foreign object 86. For instance, the recommendation can include a tool length needed to reach the foreign object 86 or an extraction feature of the tool (e.g., grasper, clamp, pliers, etc.) that are specifically suited for the foreign object's 86 size, shape, and location. In some cases, the surgeon can manually input the extraction tool 90.

The extraction plan can include an extraction path 92 relative to the medical image 80. The extraction path 92 is a path that the extraction tool 90 traverses to reach the foreign object 86. The extraction path 92 can also include the same or separate paths for the extraction tool 90 to remove the foreign object 86. The extraction path 92 may be generated to avoid interference with aspects of the surgical plan SP. Any number of extraction paths 92 can be generated. The extraction paths 92 can be linear, curved, or curvilinear. The extraction path 92 can be based in part on the recommended extraction tool 90. In one example, the extraction path 92 can be generated based on a shortest path to reach the foreign object 86 from the exterior surface of the target anatomy TA while avoiding aspects of the surgical plan SP or critical anatomical structures. In some cases, the controller can utilize bone density information to generate the extraction path 92. For example, the extraction path 92 can be generated based on minimizing the amount of dense (cortical) bone in the path 92. Any rules may be implemented by the controller, such as avoiding breach of two cortical surfaces, avoiding curved or angled paths, avoiding certain clinical approaches that are impractical or not preferred, or the like.

FIG. 3 illustrates the display 46 showing the medical image 80 of an example extraction path 92 for removing the foreign object 86 from the target anatomy TA. The medical image 80 provides visual representations of the extraction path 92 and its relation to the avoidance regions AR within the target anatomy 94. The head-mounted device HMD may also provide similar visual representations to the user. In the example of FIG. 3, the extraction path 92 represents removing the foreign object 86 from the target anatomy TA without intersecting or overlapping aspects of the surgical plan SP. Specifically, the extraction path 92 is generated relative to the femur as the foreign object 86 is located in the femur. The extraction path 92 is planned to breach the femur from above and to avoid medial/lateral approaches that could collide with the avoidance regions AR where ligaments are present. The extraction path 92 terminates and/or the controller provides an alert and/or a notification before colliding with the planned implant surface of the femur. The extraction path 92 is slightly angled due to the orientation of the foreign object 86. Hence, the illustrated extraction path 92 is intelligently generated based on a variety of clinical and practical features. Generating the extraction path 92 relative to the medical image 80 can be helpful to provide a visual representation of the possible extraction path(s) 92 as they relate to the surgical plan SP and to the patient's 12 avoidance regions AR. In other words, it is beneficial and helpful to generate the extraction plan relative to the medical image 80 so that the extraction path 92 can be visually represented and evaluated to account for any potential complications. The surgeon can review the potential extraction path(s) 92 and choose the most clinically appropriate path. In some cases, the controller can generate a score for every recommended extraction path 92. The score can be based on, or convey, accuracy, path distance, path risk, or the like.

The extraction plan can also include generating a representation of an axis 88 (shown in FIG. 3) of the foreign object 86 relative to the medical image 80 for guiding a surgeon to remove the foreign object 86 with the extraction tool 90 relative to the axis 88. The extraction path 92 may be influenced based on the axis 88 of the foreign object 86. For example, the axis 88 may provide insight into the trajectory that was taken during the installation of the foreign object 86. For example, if the foreign object 86 is a surgical screw, the axis 88 would represent a projection going through the center of the head of the surgical screw and continuing through the threaded body. The path used for installation of the surgical screw may be the same path, in reverse, as removing the surgical screw from the target anatomy TA. The axis 88 of the foreign object 86 was used to determine the angle of the extraction path 92 in the example of FIG. 3.

As shown in FIG. 3, the extraction plan may also include generation of virtual boundaries 68. The virtual boundaries 68 help ensure that in the process of removing the foreign object 86 from the target anatomy TA, the extraction tool 90 does not inadvertently intersect with any aspect of the surgical plan SP, such as avoidance regions AR. The virtual boundaries 68 can conform to the shape of the extraction path 92 and/or be sized based on the selected extraction tool 90. For example, in FIG. 3, virtual boundaries 68 are defined relative to and surround the extraction path 92. For instance, the virtual boundary 68 may be a 3D cylindrical object. Virtual boundaries 68 can be open or closed virtual walls, avoidance regions, keep in zones, keep out zones, or the like. The virtual boundaries 68 can have any suitable shape or size. The virtual boundaries 68 can be configured to be sensitive to interaction with the extraction tool 90 and/or the foreign object 86 itself.

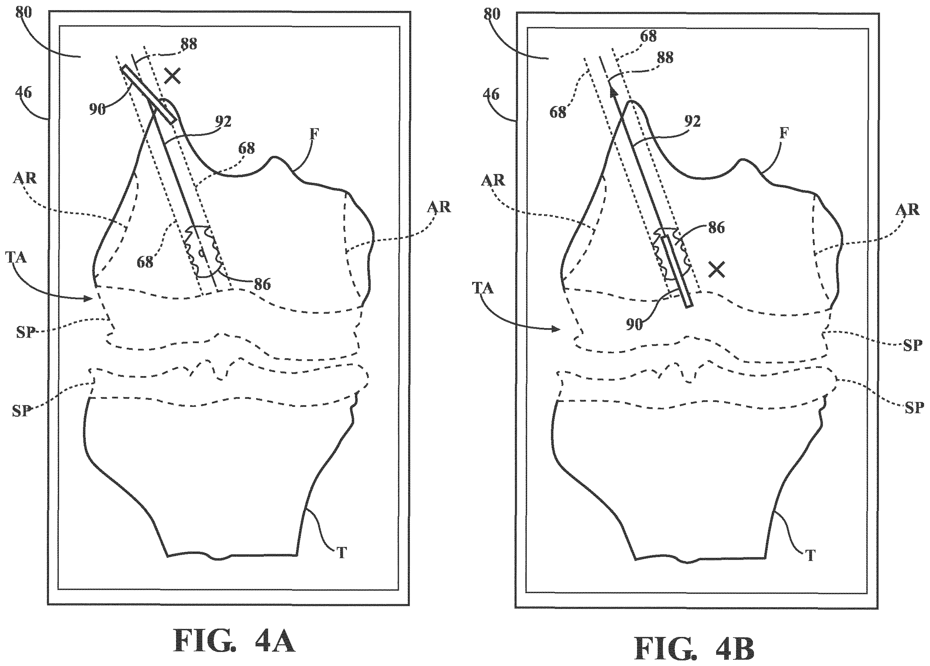

FIGS. 4A and 4B provide examples of interaction between the extraction tool 90, the extraction path 92, the foreign object 86, the target anatomy TA and virtual boundaries 68. In these examples, the content can be presented on any of the described display devices for surgical guidance. The content can be displayed as a graphical user interface. The navigation system 40 can track poses of the target anatomy TA and the extraction tool 90. Graphical representations of the extraction tool 90, the extraction path 92, the foreign object 86, the target anatomy TA and virtual boundaries 68 are displayed. A relative relationship between the representations of the extraction tool 90 and the target anatomy TA is updated based on the navigation system 40 tracking the poses of the target anatomy TA and the extraction tool 90. The controller is configured to provide the real-time relative relationships for assisting the surgeon to follow the extraction path 92 with the extraction tool 90 for extracting the foreign object 86 from the target anatomy TA. The graphical user interface can provide visual alerts or graphical indicators in response to certain conditions, such as interaction between any of the extraction tool 90, the extraction path 92, the foreign object 86, the target anatomy TA and virtual boundaries 68. The guidance provided can assist with surgeon or tool compliance with the extraction plan.

In FIG. 4A, the extraction tool 90 has deviated slightly from the extraction path 92 and has crossed the virtual boundary 68. The surgeon is immediately alerted of the virtual boundary 68 interaction by the graphical user interface visually presenting an alert (shown as an X). The visual alert can be temporary color changes to the represented extraction tool 90 or virtual boundary 68. An audible alert can simultaneously be generated by the display device. In other scenarios, if the extraction tool 90 is an active device and tracked, the extraction tool 90 can be controlled in response to virtual boundary 68 interaction. For instance, in response to virtual boundary 68 interaction, the extraction tool 90 can provide haptic, audio, visual feedback, temporarily stop moving, slow movement, or change position or orientation. Such techniques can be used with the manipulator 14 and/or robotic hand-held tools, as described above. The manipulator 14 can follow this extraction path 92 in any mode of operation, such as a manual mode or semiautomated mode of operation.

FIG. 4B illustrates the display 46 showing the medical image 80 in FIG. 4A at a second moment in time, wherein the extraction tool 90 has crossed the virtual boundary 68 defining a depth limit to avoid intersection of the extraction tool 90 with the implant region defined by the surgical plan SP. The extraction tool 90 has overshot the foreign object 86 and has gone too far into the patient's 12 bone. This may impede the eventual installation of the surgical implant as specified by the surgical plan SP. As such, by providing intraoperative guidance and feedback, the extraction tool 90 can avoid inadvertently damaging the bone required in the surgical plan SP and avoid compromising the future installation of the implant.

The extraction plan can also include generating a suggested workflow for removing the foreign object 86. The suggested surgical workflow can include a series of steps and processes that guide the surgical team from preoperative planning to post-operative care involved with removal of the foreign object 86. The suggested workflow can be designed to ensure that steps of the surgical process are carried out efficiently and effectively, with the goal of achieving the best possible outcome for the patient 12. The suggested workflow may also include a series of steps for cutting the foreign object 86 into pieces, and each of the pieces would be subsequently removed from the target anatomy 94. Examples of suggested workflow steps include, but are not limited to: size/location of the incision for removing the foreign object 86, the appropriate time/step to remove the foreign object 86, when/where/how the extraction tool 90 should be used, workflow modifications to the surgical plan SP, and/or determinations of when to execute the extraction plan relative to the surgical plan SP. Additionally, the suggested workflow may include using HMA cameras to confirm that the foreign object 86 (or all the fragments of the foreign object 86) were successfully removed from the target anatomy 94. This confirmation may use machine vision software to detect all the fragments of the foreign object 86 that were successfully removed to verify that the entire foreign object 86 was removed from the target anatomy 94. For example, the controller may determine from the techniques described above that the foreign object 86 is located in the femur and that the surgical plan SP requires five planar cuts are required on the femur. The controller can determine that one particular femur cut provides the most optimal time and exposure to extract the foreign object 86. In response to this determination, the controller can automatically modify the surgical plan SP or generate a recommendation to modify the surgical plan SP to include the execution of the extraction plan immediately after performing the identified cut.

The extraction plan can also include suggesting a setup of objects in the operating room for removing the foreign object 86. The setup can include steps to ensure that the proper surgical equipment is configured for use during a given surgical procedure. These steps can include selection of instruments, preparation of instruments, arrangement of instruments, and post-surgery care. The selection of instruments includes the recommendation of the extraction tool 90 used for removing the foreign object 86. The suggested setup may also include the controller auto-aligning the extraction tool 90 to the desired extraction path 92. The suggested setup can also include a recommend pose of the patient 12 on the surgical table, a recommended position of the manipulator 14 and pose of the robotic arm, recommendations for aspiration or irrigation, and the like. The controller can automatically determine the optimal surgical setup and generate a recommendation regarding the same.

The extraction plan can also include suggesting a region of tissue to be removed from the target anatomy 94 to facilitate removing the foreign object 86 from the target anatomy 94. The suggested region of tissue may include the foreign object 86 itself. For example, if the foreign object 86 is a surgical screw that has become stripped, the removal of the surgical screw may include using a coring reamer that is used to drill over the surgical screw thereby removing a cylinder of bone around the surgical screw.

The techniques described herein can proactively identify and formulate extraction plans for removal of a non-native foreign object near or directly in the surgical site. These techniques identify the foreign object 86 in medical imaging data to avoid complications resulting from when such foreign objects go completed undetected during surgery. By identifying the foreign object 86, a more ideal outcome is provided for the patient 12 and the surgeon. Also, the techniques prevent surprise discoveries of such foreign objects during surgery. Using the generated extraction plan, the surgeon will know about the foreign object 86 and the optimal plan to remove the foreign object 86 ahead of time. The techniques will make the surgeon aware of what the foreign object 86 is, what surrounds the foreign object 86, and/or the optimal manner or tools to remove the foreign object 86. The extraction plan avoids having the surgeon intraoperatively improvise a plan for removal of the foreign object 86. Advantageously, the extraction plan can be generated based on interaction with a surgical plan SP. Hence, the extraction plan will have considered, ahead of time, how removal of the foreign object 86 may affect the surgical plan SP. The removal of the foreign object 86 according to the extraction plan therefore can minimize complications, such as damage to implant receiving surfaces, damage to sensitive structures (such as soft tissue or ligaments), or possible modification to the surgical plan SP. During the procedure, the surgeon need not update or recreate a surgical plan that considers the implications of the foreign object 86. As such, removal of the foreign object 86 according to the extraction plan of foreign objects can improve the clinical outcome of the patient, reduce the duration of surgery, and reduce the cost of the procedure. Other advantages beyond these will be readily understood from the detailed description and figures.

## Claims

1. A computer-implemented method (300) for evaluating a medical image (80) of a target anatomy (TA), the computer-implemented method (300) comprising automatically:
detecting (302), with an object-detection algorithm (ODA), a foreign object (86) in the medical image (80), the foreign object (86) being non-native to the target anatomy (TA);
determining (304), with the object-detection algorithm (ODA), a pose of the foreign object (86) in the medical image (80);
associating (306) a surgical plan (SP) with the medical image (80);
determining (308) an interaction between the foreign object (86) and the surgical plan (SP); and
generating (310), based on the interaction, an extraction plan for removing the foreign object (86) from the target anatomy (TA).

2. The computer-implemented method (300) of claim 1, wherein, generating (310) the extraction plan for removing the foreign object (86) further includes recommending an extraction tool (90) for removing the foreign object (86).

3. The computer-implemented method (300) of any preceding claim, further comprising automatically:
determining, with the object-detection algorithm (ODA), a geometry of the foreign object (86); and
comparing the geometry of the foreign object (86) to a database (76) of known objects.

4. The computer-implemented method (300) of claim 3, wherein:
in response to comparing the geometry of the foreign object (86) to the database (76) of known objects, further comprising automatically identifying which of the known objects in the database (76) is a best-fit match to the foreign object (86); and optionally,
automatically suggesting an extraction tool (90) for removing the foreign object (86), wherein the extraction tool (90) is associated with the known object in the database (76) that is the best-fit match to the foreign object (86).

5. The computer-implemented method (300) of any preceding claim, further comprising the object-detection algorithm (ODA) feeding the medical image (80) into a machine-learning model for classifying the foreign object (86).

6. The computer-implemented method (300) of claim 5, comprising classifying the foreign object (86) as a surgical implant, wherein classifying the surgical implant comprises performing at least one of:
identifying a type of the surgical implant;
identifying a manufacturer of the surgical implant;
identifying a bone ingrowth measurement of the surgical implant; and/or
identifying how long the surgical implant has been implanted in the target anatomy (TA).

7. The computer-implemented method (300) of any preceding claim, wherein generating the extraction plan further comprises generating the extraction plan relative to the medical image (80).

8. The computer-implemented method (300) of claim 7, wherein generating the extraction plan relative to the medical image (80) further comprises generating a representation of an axis (88) of the foreign object (86) relative to the medical image (80) for guiding a surgeon to remove the foreign object (86) with an extraction tool (90) relative to the axis (88).

9. The computer-implemented method (300) of any preceding claim, wherein determining the interaction between the foreign object (86) and the surgical plan (SP) further comprises evaluating whether the extraction plan would disrupt the surgical plan (SP).

10. The computer-implemented method (300) of claim 9, wherein:
the surgical plan (SP) relates to a surgical implant for a bone provided in the medical image (80);
determining whether the extraction plan would disrupt the surgical plan (SP) further comprises determining whether the foreign object (86) would impede installation or functionality of the surgical implant; and optionally
displaying, on a display device (46), an alert or notification in response to determining whether the foreign object (86) would impede installation of the surgical implant.

11. The computer-implemented method (300) of any preceding claim, wherein generating the extraction plan for removing the foreign object (86) further comprises generating an extraction path (92) for removing the foreign object (86), and optionally;
evaluating whether the extraction path (92) would impede installation of a surgical implant; or
generating the extraction path (92) for avoiding sensitive patient physiological structures.

12. The computer-implemented method (300) of any preceding claim, wherein generating the extraction plan further comprises:
generating a suggested surgical workflow for removing the foreign object (86); and/or
suggesting a setup of a surgical tool for removing the foreign object (86).

13. The computer-implemented method (300) of any preceding claim, wherein, detecting (302), with the object-detection algorithm (ODA), the foreign object (86) in the medical image (80) further comprises applying a radio density threshold for distinguishing the foreign object (86) from surrounding patient physiological structures in the medical image (80).

14. A non-transitory computer readable medium including instructions, which when executed by one or more processors, are configured to execute the computer-implemented method (300) of any one of claims 1-13.

15. A surgical system, comprising:
a surgical tool; and
a controller comprising a non-transitory computer readable medium including instructions, which when executed by one or more processors, are configured to execute the computer-implemented method (300) of any one of claims 1-13.
